(19) Europäisches Patentamt
European Patent Office
Office européen des brevets

(11) Veröffentlichungsnummer: **0 271 433 B1**

(12) # EUROPÄISCHE PATENTSCHRIFT

(45) Veröffentlichungstag der Patentschrift: **27.01.93**

(51) Int. Cl.$^5$: **C12P 41/00**, C12P 17/02, C07C 405/00

(21) Anmeldenummer: **87730148.1**

(22) Anmeldetag: **12.11.87**

(54) **Racematspaltung von 7 alpha-Acyloxy-6 beta-hydroxy-methyl-2-oxabicyclo 3.3.0 octan-3-onen durch stereospezifische enzymatische Acylat-Hydrolyse.**

(30) Priorität: **13.11.86 DE 3638762**

(43) Veröffentlichungstag der Anmeldung:
**15.06.88 Patentblatt 88/24**

(45) Bekanntmachung des Hinweises auf die Patenterteilung:
**27.01.93 Patentblatt 93/04**

(84) Benannte Vertragsstaaten:
**AT BE CH DE ES FR GB GR IT LI LU NL SE**

(56) Entgegenhaltungen:
**EP-A- 0 041 661**
**EP-A- 0 127 386**
**FR-A- 2 582 648**
**GB-A- 1 579 464**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Petzoldt, Karl, Dr.**
**Flachsweg 10**
**W-1000 Berlin 38(DE)**
Erfinder: **Dahl, Helmut, Dr.**
**Gollanczstrasse 102**
**W-1000 Berlin 28(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein Verfahren zur stereospezifischen Acylat-Hydrolyse racemischer 7α-Acyloxy-6β-hydroxymethyl-2-oxabicylo[3.3.0]octan-3-onen zu den entsprechenden optisch aktiven Alkoholen mit Hilfe von Enzymen.

Es eignet sich besonders zur Herstellung von optisch aktiven (-)-Oxabicyclo[3.3.0]octanolonen der Formel (-)-I,

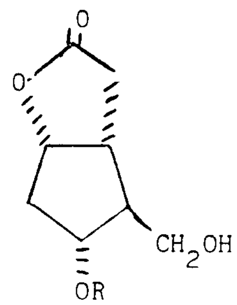

(-)-I ,

worin R Wasserstoff oder den Rest

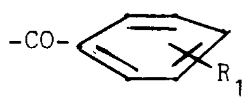

mit $R_1$
in der Bedeutung eines Wasserstoffs, Alkyls mit 1-7 C-Atomen oder Phenyls bedeuten kann.

Es ist dadurch gekennzeichnet, daß man racemische Oxabicyclo[3.3.0]octanolon-acylate der Formel (±)-II,

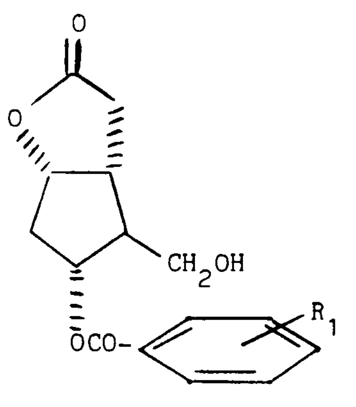

(±)-II ,

worin $R_1$ die oben angegebene Bedeutung hat, enzymatisch einer stereospezifischen Acylat-Hydrolyse unterwirft und das (-)-I (R =

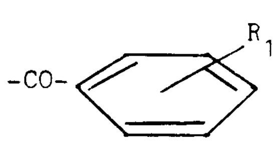

)

vom verseiften (+)-I (R = H) abtrennt oder das verseifte (-)-I (R = H) vom unverseiften (+)-II abtrennt.

Wenn $R_1$ einen Alkylrest mit 1-7 C-Atomen darstellt, sind damit die Reste Methyl, Ethyl, n-Propyl,

Isopropyl, n-Butyl, Isobutyl, sek.-Butyl, tert.-Butyl, n-Pentyl, Isopentyl, sek.-Pentyl, Neopentyl, n-Hexyl, Isohexyl, n-Heptyl, Isoheptyl usw. gemeint.

Die Verbindungen der Formel (-)-I sind wertvolle Zwischenprodukte bei der Synthese pharmakologisch wertvoller Prostaglandin- und Prostacyclin-Analoga (Übersichten z.B. von J. Bindra, R.Bindra, Prostaglandin Synthesis, Academic Press, New York 1977 und von C. Szantay, L. Novak, Sythesis of Prostaglandins, Akademiai Kiado, Budapest, 1978). Zur Synthese der zu den Naturprodukten analogen Strukturen werden die Ester dieser Enantiomeren-Reihe benötigt.

Obwohl asymmetrische Synthesen bekannt sind, wird bei der technischen Herstellung die Einführung der optischen Aktivität auf dem Weg einer Racemattrennung bei der Zwischenstufe (±)-3 bevorzugt. Hierbei sind folgende Verfahrensschritte nötig:

1. Verseifung des Lactons zur Hydroxysäure.
2. Überführung in das Gemisch diastereomerer Salze, z.B. mit d-α-Phenylethylamin.
3. Kristallisation zur Gewinnung des diastereomeren reines Salzes.
4. Überführung des Salzes in das optisch aktive Lacton (-)-3.
5. Weitere Umsetzung zur optisch aktiven Vorstufe (-)-I.

Durch die Neigung der Hydroxysäure, das Lacton wieder zurückzubilden, bevor eine optische Aufreinigung erfolgen konnte, sowie durch Ausbeuteverluste beim Kristallisieren, hat sich das nachstehende Verfahren nicht bewährt.

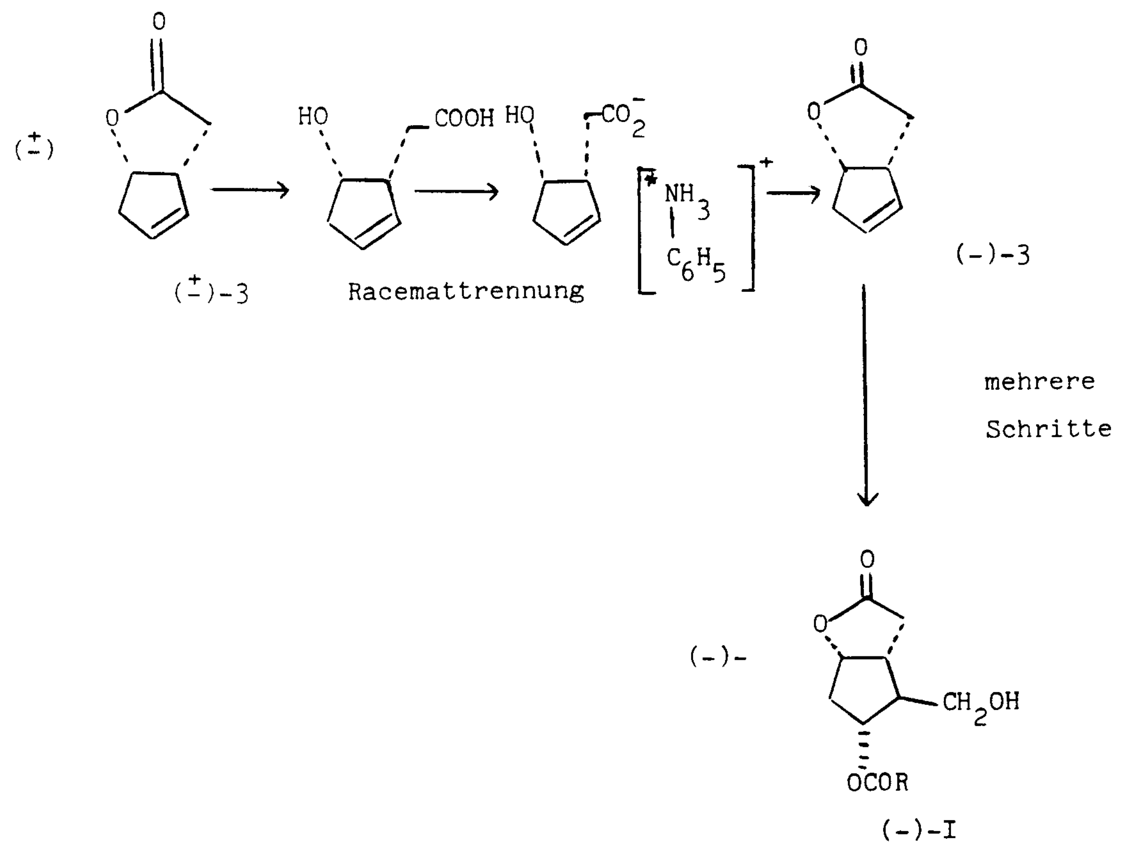


(±)-3 (-)-3 (-)-I

Bei der vorliegenden Erfindung wird die Trennung auf der Stufe der racemischen 7α-Acyloxy-6β-hydroxymethyl-2-oxa-bicyclo[3.3.0]octan-3-one der Formel (±)-II durch enzymatische Verseifung vorgenommen, die zu einem Produktgemisch führt, aus dem die gewünschten Verbindungen der Formel (-)-I entweder durch Extraktion oder aus der wäßrigen Phase zum Beispiel durch Säulenchromatographie erhalten werden können.

Die Ausgangsverbindungen lassen sich nach GB 1 579 464 herstellen.

Die Verwendung der nach dem erfindungsgemäßen Verfahren erhaltenen optisch aktiven Ester der

Formel (-)-I

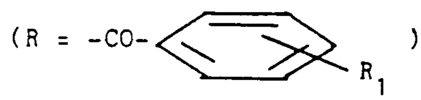

als Zwischenprodukte für die Herstellung von pharmakologisch wertvolle Prostaglandin- und Prostacyclin-Analoga geht aus den bereits genannten Übersichtsartikeln hervor.

Das ebenfalls erhältliche optisch aktive Diol der Formel (-)-I (R = H) kann besonders dann Verwendung finden, wenn bei der Folgesynthese zunächst die primäre Hydroxylgruppe geschützt werden muß und die sekundäre Hydroxylgruppe freibleiben kann. Dies ist z.B. bei der Synthese von Carbacyclin-Zwischenprodukten der Fall, wie sie in EP 41 661 beschrieben wird.

Als Enzyme eignen sich für das erfindungsgemäße Verfahren vorzugsweise
Subtilisin aus Bacillus subtilis (Fa. Boehringer, Mannheim)
Lipase " Sclerotinia" (Fa. Nagase)
Alkaline Proteinase (Fa. Nagase)
Protease aus Bacillus subtilis
Alcalase T (Fa. NOVO)

Die Enzyme können sowohl in gelöster, suspendierter oder immobilisierter Form (z.B. an BrCN-aktivierter Sepharose oder an Oxiranacrylperlen oder in irgendeiner anderen immobiliserten Form) eingesetzt werden.

Die nach dem erfindungsgemäßen Verfahren herstellbaren optisch aktiven Oxabicyclooctanolon-Derivate der Formel (-)-I sind wertvolle Zwischenprodukte für die Synthese pharmakologisch wirksamer Prostaglandine und Prostacycline. Es zeigte sich, daß der größere Teil der Stereospezifisch hydrolysierenden Enzyme die racemischen Verbindungen der Formel (±)-II zum optisch aktiven Diol der Formel (+)-I (R = H) verseift, wobei die in ihrer absoluten Konfiguration dem natürlichen Prostaglandin $PGF_{2\alpha}$entsprechende Komponente des eingesetzten Racemats (±)-II unverseift liegen bleib

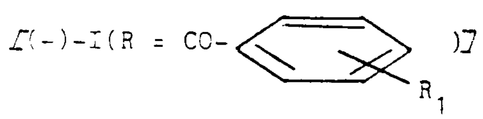

und nach Abtrennung vom Diol zur Synthese natürlich konfigurierter Prostaglandin-Analoga eingesetzt werden kann.

Andere Enzyme wiederum verseifen von den beiden Komponenten des eingesetzten Racemats (±)-II die natürlich konfigurierte Form zum Diol (-)-I(R = H), während das "falsche", unnatürlich konfigurierte Enantiomere unverseift bleibt. In diesem Falle wird das Diol für die Synthese $PGI_2$-analoger Prostacycline herangezogen. Das erfindungsgemäße Verfahren arbeitet ansonsten unter an sich bekannten Bedingungen, die für enzymatische Reaktionen bekannt sind. Der Ablauf der enzymatischen Umwandlung wird durch Analyse laufend entnommener Proben verfolgt. Als Analysenmethoden eignen sich HPLC oder dünnschichtchromatographische Schnellanalysen (Kieselgelplatten von Merck/Darmstadt, Entwicklung mittels Methylenchlorid/Aceton 1:1 und Anfärben mit Schwefelsäure).

Die Reaktion wird abgebrochen und der Ansatz aufgearbeitet, sowie 50 % des eingesetzten racemischen Substrates umgesetzt sind.

Das erfinderische Verfahren eignet sich besonders zur stereospezifischen Verseifung folgender Prostaglandin-Zwischenstufen:

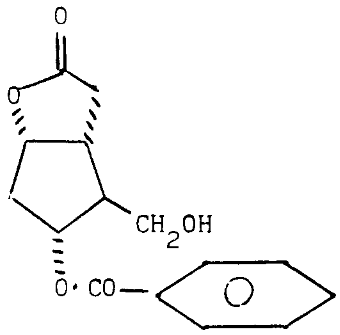

(±)-1

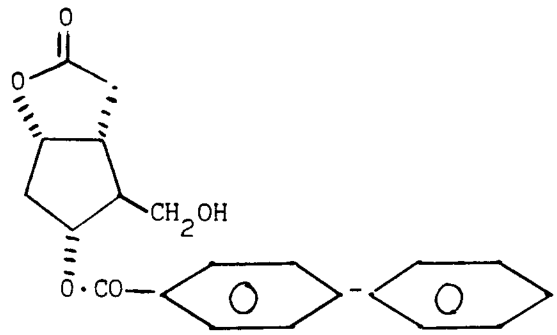

(±)-2

Die folgenden Ausführungsbeispiele dienen zur Erläuterung des erfindungsgemäßen Verfahrens.

Beispiel 1

300 mg (±)-7α-Benzoyloxy-6β-hydroxymethyl-2-oxabicyclo [3.3.0] octan-3-on werden in 9 ml Ethanol gelöst und mit einer Lösung von 750 mg Alkaline Proteinase (Fa. Nagase) in 100 ml 0,1 M Phosphatpuffer pH7 vereinigt. Die Lösung wird bei 28° C auf einem Rotationsschüttler geschüttelt, wobei der Ablauf der Reaktion durch Analyse laufend entnommener Proben verfolgt wird. Nach 19 Stunden Reaktionszeit sind 50 % des eingesetzten Substrates umgewandelt. Der Ansatz wird nun 3 mal mit je 50 ml Methylisobutylketon extrahiert, wobei das unumgesetzte Benzoat in die MiBK-Phase wandert, während die verseifte Verbindung in der wässrigen Phase verbleibt.

Die MiBK-Extrakte werden vereinigt und im Vakuum zur Trockne eingeengt. Der verbliebene Rückstand wird aus Aceton umkristallisiert. Man erhält 135 mg (-)-7α-Benzoyloxy-6β-hydroxymethyl-2-oxabicyclo [3.3.0] octan-3-on vom Schmelzpunkt 116-117° C und einem Drehwert von$[\alpha]_D^{20}$ -80,5° (c =1,050 in $HCCl_3$).

Beispiel 2

300 mg (±)-7α-Benzoyloxy-6β-hydroxymethyl-2-oxabicyclo [3.3.0] octan-3-on werden in 100 ml 0,1 M Phsophatpuffer pH 7 suspendiert, 750 mg Subtilisin aus Bacillus subtilis (Fa. Boehringer, Mannheim) zugefügt und die Mischung mit einem Ultra-Turrax homogenisiert. Die Suspension wird anschließend 16 Stunden bei 28°C auf einem Rotationsschüttler geschüttelt, wonach 50% des eingesetzten racemischen Substrates verseift sind. Der Ansatz wird nun im Wasserstrahlvakuum zur Trockne eingeengt, der verbliebene Rückstand 3 mal mit Methanol eluiert, die vereinigten Methanol-Eluate wiederum zur Trockne gebracht und über eine Kieselgelsäure chromatographiert (Elutionsmischung Methylenchlorid-Aceton 66 + 33). Die zuerst eluierte Fraktion 1 enthält 122 mg (-)-7α-Benzoyloxy-6β-hydroxymethyl-2-oxabicyclo [3.3.0] octan-3-on vom Schmelzpunkt 114-116° C und einem Drehwert von $[\alpha]_D$-82,3° (c = 1,015 in Methanol).

Beispiel 3

Unter den Bedingungen des Beispiels 2 werden 300 mg (±)-7α-Benzoyloxy-6β-hydroxymethyl-2-oxabicyclo[3.3.0]octan-3-on in Phosphatpuffer pH7 mit 750 mg Lipase Sclerotina (Fa. Nagase) 64 Stunden bei 28° C behandelt. Nach säulenchromatographischer Abtrennung des verseiften unnatürlich konfigurierten Enantiomeren erhält man 115 mg (-)-7α-Benzoyloxy-6β-hydroxymethyl-2-oxabicyclo[3.3.0]octan-3-on vom Schmelzpunkt 115-116° C und einem Drehwert von $[\alpha]_D$-79,8° (c = 1,02 in Methanol).

Beispiel 4

Unter den Bedingungen des Beispiels 2 werden 300 mg (±)-7α-Benzoyloxy-6β-hydroxymethyl-2-oxabicyclo[3.3.0]octan-3-on in Phosphatpuffer pH7 mit 750 mg Alcalase T (Fa. NOVO) 40 Stunden bei 28° C hydrolysiert. Nach säulenchromatographischer Auftrennung des Reaktionsgemisches erhält man 142 mg

(-)-7 α-Benzoyloxy-6β-hydroxymethyl-2-oxabicyclo[3.3.0]octan-3-on vom Schmelpunkt 112-114° C und einem Drehwert von$[\alpha]_D$-77,1° (c = 1,005 in Methanol).

Beispiel 5

Unter den Bedingungen des Beispiels 2 werden 300 mg (±)-7α-Benzoyloxy-6β-hydroxymethyl-2-oxabicyclo[3.3.0]octan-3-on in Phosphatpuffer pH 7 mit 750 mg Protease aus Bacillus subtilis 15 Stunden bei 28°C behandelt. Nach säulenchromatographischer Auftrennung des Reaktionsgemisches erhält man 119 mg (-)-7α-Benzoyloxy-6β-hydroxymethyl-2-oxabicyclo[3.3.0]octan-3-on vom Schmelzpunkt 113-115° C und einem Drehwert von$[\alpha]_D$ - 78,9° (c = 1,025 in Methanol).

**Patentansprüche**

**1.** Verfahren zur Herstellung von optisch aktiven (-)-Oxabicyclo [3.3.0]octanolonen der Formel (-)-I,

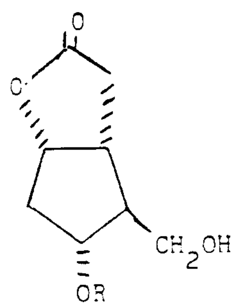

(-)-I ,

worin R Wasserstoff oder den Rest

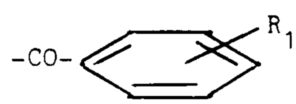

mit
$R_1$ in der Bedeutung eines Wassertoffs, Alkyls mit 1-7 C-Atomen oder Phenyls bedeuten kann, dadurch gekennzeichnet, daß man ein racemisches Oxabicyclo[3.3.0]octanolon-acylat der Formel (±)-II,

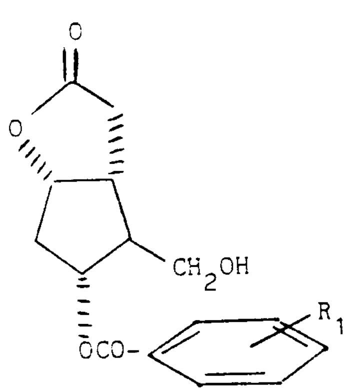

(±)-II ,

worin $R_1$ die oben angegebene Bedeutung hat, enzymatisch einer stereospezifischen Acylat-Hydrolyse unterwirft und das (-)-I (R =

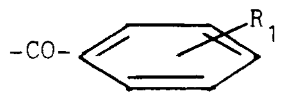 )

vom verseiften (+)-I (R = H) abtrennt oder das verseifte (-)-I (R = H) vom unverseiften (+)-II abtrennt.

2. Verfahren nach Anspruch 1, dadurch gekennzeichnet, daß man als Enzym Subtilisin aus Bacillus subtilis, Lipase Sclerotinia, Alkaline Proteinase, Protease ausBacillus subtilis oder Alcalase T verwendet.

## Claims

1. Process for the preparation of optically active (-)-oxabicyclo[3.3.0]octanolones of the formula (-)-I

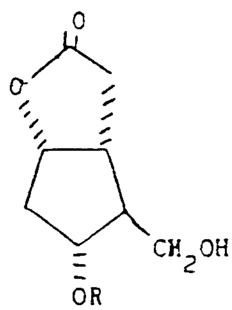

(-)-I

wherein R represents hydrogen or the radical

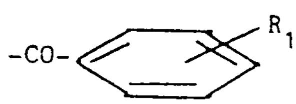

wherein $R_1$ may represent hydrogen, alkyl having from 1 to 7 carbon atoms or phenyl, characterised in that a racemic oxabicyclo[3.3.0]octanolone acylate of the formula (±)-II

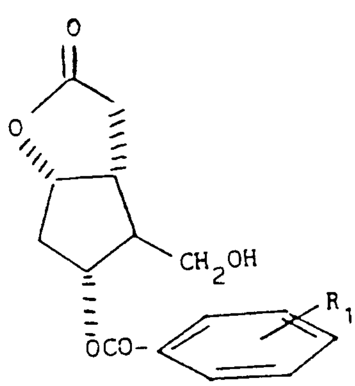

(±)-II,

wherein $R_1$ is as defined above, is subjected enzymatically to a stereospecific acylate hydrolysis and the (-)-I

(R = 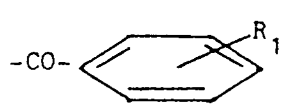 )

is separated from the hydrolysed (+)-I (R = H) or the hydrolysed (-)-I (R = H) is separated from the unhydrolysed (+)-II.

2. Process according to claim 1, characterised in that there is used as enzyme subtilisin from Bacillus subtilis, lipase Sclerotinia, alkaline proteinase, protease from Bacillus subtilis or Alcalase T.

**Revendications**

1. Procédé pour préparer des oxabicyclo[3.3.0]octanolones (-) optiquement actives répondant à la formule (-)-I :

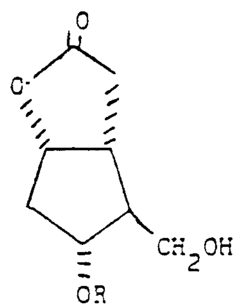

(-)-I

dans laquelle R représente l'hydrogène ou un radical

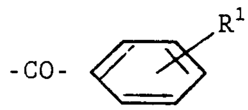

dont le symbole $R_1$ représente l'hydrogène, un alkyle contenant de 1 à 7 atomes de carbone ou un phényle, procédé caractérisé en ce qu'on soumet un acylate d'oxabicyclo[3.3.0]octanolone racémique répondant à la formule (±)-II :

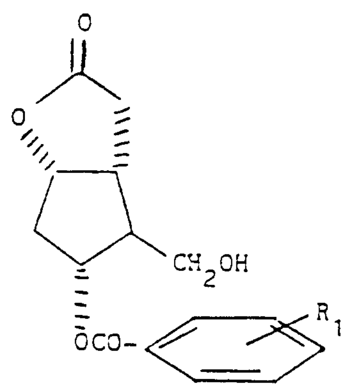

(±)-II

dans laquelle $R_1$ a la signification indiquée ci-dessus, à une hydrolyse d'acylate stéréospécifique par voie enzymatique, et on sépare le composé (-)-I

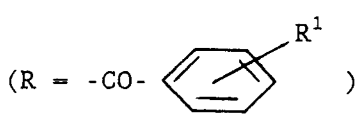

du composé (+)-I saponifié (R = H), ou on sépare le composé saponifié (-)-I (R = H) du composé

(+)-II non saponifié.

**2.** Procédé selon la revendication 1 caractérisé en ce qu'on utilise, comme enzyme, la subtilisine de Bacillus subtilis, la lipase Sclerotinia, la Protéinase alcaline, la protéase de Bacillus subtilis ou l'Alcalase T.